# FASCICULE DE BREVET EUROPEEN

(11) **EP 2 516 390 B1**
(45) Date de publication et mention de la délivrance du brevet: **26.02.2014**
(21) Numéro de dépôt: 10809306.3
(22) Date de dépôt: 21.12.2010
(51) Int. Cl.: C07C 333/20, C07D 295/21

(54) **PROCEDE DE PREPARATION DE DITHIOCARBAMATES NOTAMMENT A PARTIR DE POLYOLS DU TYPE GLYCEROL**
VERFAHREN FÜR DIE ZUBEREITUNG VON DITHIOCARBAMATEN, IM BESONDEREN AUS GLYCEROL-POLYOLEN
METHOD FOR PREPARING DITHIOCARBAMATES IN PARTICULAR FROM POLYOLS OF THE GLYCEROL TYPE

(30) Priorité: 21.12.2009 FR 0906203
(43) Date de publication de la demande: 31.10.2012
(73) Titulaire: Centre National de la Recherche Scientifique, 75794 Paris Cedex 16 (FR); Université De Poitiers, 86034 Poitiers Cedex (FR)
(72) Inventeur: JEROME, François, F-86800 Sevres Anxaumont (FR); DE SOUSA, Rodolphe, F-86440 Migne-auxances (FR); BARRAULT, Joël, F-86240 Liguge (FR); POUILLOUX, Yannick, F-86550 Mignaloux-beauvoir (FR)
(74) Mandataire: Vaillant, Jeanne
(86) Numéro de dépôt international: PCT/FR2010/052867
(87) Numéro de publication internationale: WO 2011/083255

(56) Documents cités:
- US-A- 3 407 222
- C. LEN, ET AL.: "Synthesis of carbamic esters derivatives of itols: antifungal activity against various crop diseases", JOURNAL OF AGRICULTURAL AND FOOD CHEMISTRY, vol. 45, no. 1, 20 janvier 1997 (1997-01-20), pages 3-6, XP002593803, AMERICAN CHEMICAL SOCIETY, WASHINGTON, DC, US ISSN: 0021-8561, DOI: 10.1021/jf9607371 cité dans la demande

## Description

La présente invention concerne un nouveau procédé de préparation de dithiocarbamates, ainsi que leurs utilisations notamment phytosanitaires.

### Domaine de l'invention.

Les dithiocarbamates constituent une large famille chimique et sont utilisés dans la chimie du caoutchouc, comme accélérateurs de vulcanisation, ou pour contrôler les polymérisations radicalaires, comme inhibiteurs d'enzymes, présentant d'importants effets sur de nombreux systèmes biologiques, et dans les domaines phytosanitaires et pharmaceutiques. La famille des dérivés de l'acide dithiocarbamique comporte notamment des dérivés du zinc, du fer, du sodium et du manganèse utilisés comme pesticides et/ou fongicides, pour les cultures maraîchères, les céréales, les vignes, par exemple. Toutefois, des problèmes toxicologiques (maladies associées) et environnementaux, comme la contamination des sols et nappes phréatiques, découlent de l'utilisation intensive de ces pesticides, toxiques et faiblement biodégradables. Leur métabolisation peut en effet libérer des composés très toxiques, notamment de l'éthylènethiourée reconnue responsable de cancers de la thyroïde et de la maladie de Parkinson. Compte tenu de leur toxicité et de celle de leurs métabolites, il s'est développé un intérêt pour substituer les métaux par des polyols, notamment des polyols naturels, comme le glycérol et les sucres. La dérivatisation des dithiocarbamates avec des molécules hydrocarbonées, notamment avec les polyols^{1 à 6} rend les dithiocarbamates solubles dans l'eau et permet aussi de contourner l'utilisation intempestive de métaux.
Les dithiocarbamates de glycérol ou glycérols fonctionnalisés par une fonction dithiocarbamate présentent un intérêt particulier du fait de leur plus grande biocompatibilité et des tests biologiques montrent leurs propriétés pesticides. Ainsi, certains dithiocarbamates de glycérol (DTCGs) se sont montrés prometteurs, selon certaines études^{1 à 4}, par des propriétés antifongiques proches de celles du manèbe et du sel de sodium de l'acide diéthyldithiocarbamique. Certains DTCGs sont utilisés en médecine pour le traitement de cancers. La famille des DTCGs est de plus une famille de molécules importantes pour le piégeage des métaux.

Ils sont usuellement synthétisés par des voies classiques de la chimie organique, en plusieurs étapes, produisant de nombreux déchets et avec des rendements globaux faibles.

### Art antérieur

Ces molécules sont en général synthétisées sous forme de sels qui assurent leur solubilité dans l'eau, selon le schéma 1 suivant, par substitution du métal par un polyol naturel (A) ou par des voies classiques à partir de produits synthétiques (B)

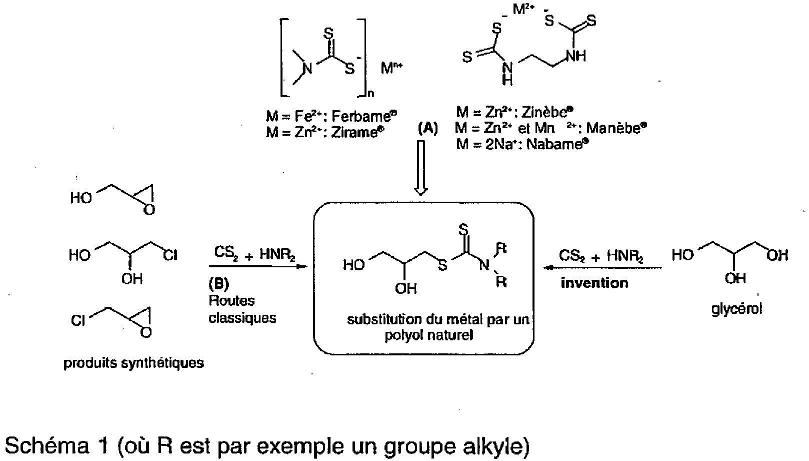

Du point de vue de la synthèse, l'introduction directe du dithiocarbamate sur le glycérol est complexe. La substitution nucléophile d'un des groupes hydroxyle du glycérol est difficile et on préfère généralement employer des halogénures d'alkyle plutôt que des alcools, mais ces voies produisent une quantité stoechiométrique de sel.
Il est connu d'utiliser une méthode impliquant les alcools et mettant en oeuvre un réactif de Mitsunobu, et celui-ci génère une quantité stoechiométrique de déchet. De plus, à partir du glycérol qui comporte trois groupes hydroxyle, des produits polysubstitués se forment, et on doit recourir à des agents de protection et de déprotection, ce qui complexifie la purification.
Pour ces raisons, le glycérol naturel n'est pas utilisé pour la production de DTCGs, mais plutôt des composés plus réactifs comme le glycidol, l'épichlorhydrine ou le 3-chloropropane-1,2-diol, selon des voies classiques (B) (cf schéma 1).

Ainsi, Rufin et al.¹ décrivent la synthèse d'esters carbamiques possédant un squelette carbohydraté dérivé du glycérol ou du D-glucose et deux groupes *N,N-*diéthyldithiocarbamoyle et d'esters bisdithiocarbamiques possédant des fonctions cétone ou ester d'alkyle. Les bis-dithiocarbamates de glycérol sont obtenus à partir de 1,3-dichloro-1,3-dideoxyglycérol et de sels de lithium de l'acide *N,N-*diéthyldithiocarbamique, dans l'acétone à reflux. Leur activité anti-fongique vis-à-vis de *Fusarium oxysporum* a été évaluée et comparée au manèbe et au sel de sodium de l'acide *N,N*-diéthyldithiocarbamique.
Len et al.² ont décrit la synthèse d'esters carbamiques présentant un squelette itol et un ou deux groupes thiocarbamoyles à partir du 2,3-O-isopropylidène-DL-glycérol ou du 1-chloro-1-deoxyglycérol, et leur activité antifongique sur *Alternaria brassicae, Pseudocercosporella herpotrichoides, Septoria nodorum et Phytophtora cinnamomi,* comparée à celle du carbendazime et du manèbe.
Len et al.³ ont décrit une synthèse de bis-dithiocarbamates de glycérol à partir de 1,3-dichloro-1,3-dideoxyglycérol et de sels de lithium de l'acide *N,N-*diéthyldithiocarbamique, dans l'acétone à reflux, et leur activité anti-fongique sur les mêmes espèces, comparée à celle du carbendazime et du manèbe.
Len et al.⁴ ont décrit une synthèse d'esters carbamiques en quatre étapes à partir du 1,2-5,6-di-*O*-isopropylidène-α-D-glucofuranose, ainsi que leur activité anti-fongique sur les mêmes espèces, comparée à celle du carbendazime et du manèbe.
Len et al. ^{5,6} ont décrit la bis-carbamoylation régiospécifique de D-glucose protégé (di-*O*-isopropylidène-D-glucose) par un procédé en quatre étapes qui met en oeuvre le composé protégé 3-iodé-6-*O*-tosylé.
Le document FR 2 735 130 décrit la synthèse de dithiocarbamates de polyols où on utilise de l'épichlorhydrine ou du 1,3-dichloro-2-propanol, onéreux et toxiques, mis en réaction avec des sels dithiocarbamiques en présence de solvants organiques peu respectueux de l'environnement (diméthylsulfoxyde, toluène). La réaction est peu sélective et génère des quantités importantes de sels dont l'élimination malaisée augmente le coût économique et environnemental de la synthèse.
Les synthèses connues évoquées ci-dessus mettent en oeuvre des produits chimiques souvent toxiques, chers et susceptibles de générer beaucoup de déchets.

Il existe donc un besoin pour une méthode de synthèse de DTGCs, composés dithiocarbamate, notamment fongicides et/ou pesticides, qui puissent être mise en oeuvre de façon simple, en peu d'étapes, méthode peu coûteuse et qui ait moins d'impact sur l'environnement que les méthodes usuelles, notamment qui ne mette pas en oeuvre de solvant organique nuisible, qui présente de bons rendements, une bonne régiosétectivité et une bonne chimiosélectivité, dont les sous-produits peuvent être facilement éliminés ou réutilisés, et qui puisse être réalisée à partir de produits de départ peu onéreux et abondants.

La Demanderesse a maintenant mis au point un procédé de synthèse de dithiocarbamate ou bis-dithiocarbamates de glycérol (ci-après DTCGs) dans lequel, dans un milieu réactionnel comportant (1) un carbonate, choisi parmi le diéthylcarbonate, le diméthylcarbonate, le carbonate d'éthylène et le carbonate de propylène, associé à un polyol du type glycérol en présence d'un catalyseur basique ou (2) un carbonate cyclique ou polycarbonate cyclique à cinq chaînons, en milieu solvant, on ajoute une amine primaire ou secondaire en présence de sulfure de carbone, et on récupère le DTCG formé.

Dans un premier mode de réalisation, l'invention concerne un procédé de préparation de dithiocarbamates de glycérol (DTCGs) dans lequel un polyol du type glycérol est mis en présence d'un carbonate choisi parmi le diéthylcarbonate, le diméthylcarbonate, le carbonate d'éthylène ou le carbonate de propylène, en présence d'un catalyseur basique, puis on ajoute au milieu réactionnel une amine primaire ou secondaire en présence de sulfure de carbone, et on récupère le DTCG formé.
Dans un second mode de réalisation, un carbonate cyclique à cinq chaînons, en milieu solvant, est mis en réaction avec une amine primaire ou secondaire et permet d'obtenir les DTCGs en une seule étape. Entre autres avantages de cette variante, on peut citer la très haute pureté des DTCGs obtenus ainsi que des rendements très élevés.

Les voies de synthèse selon l'invention sont différentes de celles qui sont décrites dans FR 2 735 130 et les articles cités ci-dessus, par la stratégie de synthèse utilisée et par les substrats employés. Le procédé selon la présente invention présente, entre autres les avantages suivants. Conformément à l'invention, il est maintenant possible de synthétiser des DTCGs en substituant, dans des conditions particulièrement douces et de façon hautement sélective, un groupe hydroxyle du glycérol ou d'un polyol du type glycérol par un groupe dithiocarbamate. Cette voie de synthèse s'inscrit dans le concept de « chimie verte ». Elle est économe en atome et ne produit pas ou peu de rejet soufrés ou azotés et pas de métal ni de sel.
Il s'agit de synthèses de divers DTCGs en une seule étape, directement à partir du glycérol, dérivé naturel, non-toxique, abondant, biodégradable et peu onéreux, et même à partir d'autres polyols du type glycérol, notamment d'autres diols, associé à un carbonate, ou encore directement à partir de carbonates à cinq chaînons. La voie de synthèse n'est en effet pas réservée au seul glycérol : d'autres polyols comportant le motif -CH(OH)-CH₂OH, tels que le propanediol et l'éthylèneglycol, peuvent être couplés à un carbonate, à une amine et au sulfure de carbone pour donner les esters de dithiocarbamate correspondants. Des carbonates à cinq chaînons, en présence d'une amine et de sulfure de carbone donnent aussi directement les DTCGs correspondants.
L'invention ouvre la voie à des synthèses qui présentent notamment les avantages suivants. Elles sont conformes à la protection de l'environnement et économiquement viables, les produits de départ étant peu onéreux et abondants, comme le glycérol qui est un co-produit de l'industrie des huiles végétales et du biodiesel, convertis en produits à haute valeur ajoutée. Les produits synthétisés peuvent être considérés comme des produits plus sûrs que les produits pesticides, fongicides ou de façon générale les produits phytosanitaires usuels.
De plus, les résultats de la synthèse à grande échelle sont conformes aux résultats obtenus en laboratoire.
Le procédé de la présente invention permet non seulement l'introduction directe et en une seule étape d'un groupe dithiocarbamate sur la molécule du polyol selon la variante préférée, il permet également d'activer régiosélectivement et chimiosélectivement le glycérol, voire d'autres polyols. Le procédé de l'invention est une alternative avantageuse aux procédés enzymatiques qui sont jusqu'ici les seuls qui permettent de contrôler précisément la sélectivité des réactions impliquant notamment le glycérol.

La présente invention concerne également l'utilisation d'un polyol du type glycérol ou du glycérol comme solvant dans un procédé selon l'invention.

Comme cela apparaîtra dans la description détaillée et dans les exemples, le procédé selon l'invention répond aux divers problèmes évoqués ci-dessus. D'autres avantages et caractéristiques de l'invention apparaîtront à la lecture de la description détaillée suivante, dans les exemples, et au vu des figures annexées.
La figure 1 représente un schéma d'un procédé de l'invention mis en oeuvre selon la première variante, mise en oeuvre avec du glycérol et du diéthylcarbonate, NaOH, CS₂ et NH(C₂H₅)₂, et les recyclages qui ont été réalisés.
La figure 2 présente les résultats du recyclage de la phase glycérol/NaOH, dans un procédé selon l'invention notamment celui de la figure 1.

Le schéma 2 ci-dessous présente la voie de synthèse selon la première variante qui comporte la carbonatation du polyol suivie de la réaction avec l'amine et le CS₂. Il s'agit d'un procédé dit « one pot-two steps »

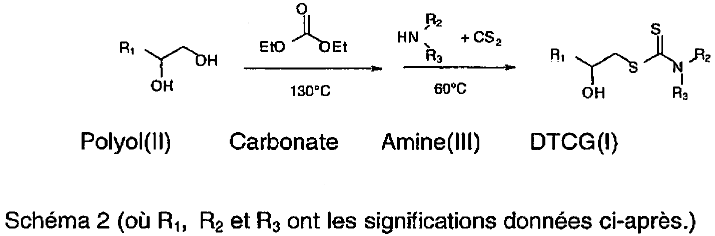

Dans la variante illustrée ci-dessus avec le diéthylcarbonate, le carbonate peut être aussi choisi parmi le diéthylcarbonate, le diméthylcarbonate et le carbonate d'éthylène ou le carbonate de propylène.

Toutefois, dans un mode de réalisation particulier, le procédé peut être mis en oeuvre directement à partir d'un carbonate cyclique, selon le schéma 3 ci-dessous 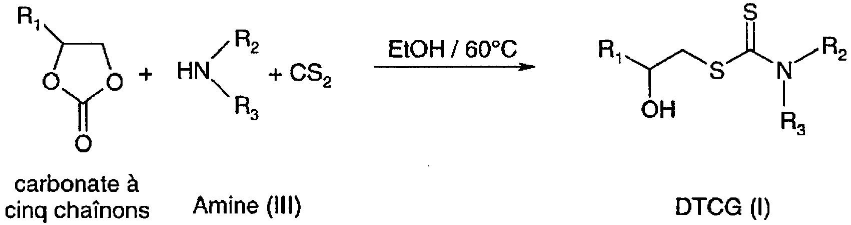 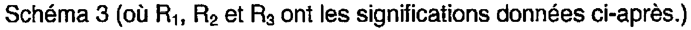 ou d'un carbonate cyclique à cinq chaînons selon le schéma 3' ci-dessous, 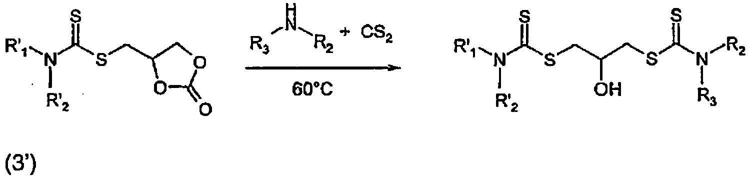 où R'₁ et R'₂ ont les mêmes significations que R₂ et R₃, significations données ci-après,
ou encore d'un polycarbonate cyclique à cinq chaînons selon le schéma 3 " ci-dessous 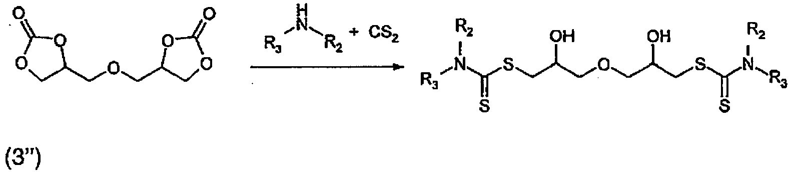 où R₂ et R₃ ont les significations données ci-après.
Plus précisément, l'invention concerne un procédé de préparation de DTCGs de formule (I)

(I) R₁-CH(OH)-CH₂-S-C(S)-NR₂R₃

dans laquelle
- R₁ représente H ;
   un radical alkyle en C₁-C₂₂, linéaire ou ramifié ou cycloalkyle en C₃-C₁₆, non-substitué ou substitué par un (ou plusieurs) groupe(s) hydroxy, -O-alkyle, thiohydroxy ou -S-alkyle, la chaîne du radical alkyle pouvant être interrompue par un ou plusieurs hétéroatomes O, N ou S ;
   un radical alcènyle en C₂-C₂₂, linéaire ou ramifié, mono- ou poly-insaturé;
   un radical alcynyle en C₂-C₆;
   un radical aryle en C₆-C₁₀; ou
   un radical arylalkyle en C₇ à C₂₂;
   et
- R₂ et R₃, indépendamment l'un de l'autre, représentent H ;
   un radical alkyle en C₁-C₂₂, linéaire ou ramifié ou cycloalkyle en C₃ à C₁₆ non-substitué ou substitué par un (ou plusieurs) groupe(s) hydroxy, -O-alkyle, thiohydroxy ou -S-alkyle, la chaîne du radical alkyle pouvant être interrompue par un ou plusieurs hétéroatomes O, N ou S ;
   un radical alcènyle en C₂-C₂₂, linéaire ou ramifié, mono- ou poly-insaturé;
   un radical alcynyle en C₂-C₆;
   un radical aryle en C₆-C₁₆; ou
   un radical arylalkyle en C₇ à C₂₂; ou
   R₂ et R₃ ensemble avec l'atome d'azote auquel ils sont liés forment un groupe cycloamino.
Dans un mode particulier, elle concerne les composés bis-dithiocarbamates de glycérol de formule

(I') R'₁R'₂N-CS-S-CH₂-CH(OH)CH₂-S-CS-NR₂R₃

ou de formule

(I") (R₂R₃N-CS-S-CH₂-CH(OH)CH₂)₂O

où R'₁ et R'₂ ont les mêmes significations que R₂ et R₃ ci-dessus.
Selon ce procédé, dans une première variante, on met en présence le polyol, un carbonate et un catalyseur basique et on ajoute une amine primaire ou secondaire en présence de CS₂.

Par « *alkyle* », on entend une chaîne hydrocarbonée, linéaire ou ramifiée, de 1 à 22 atomes de carbone, de préférence 1 à 12 atomes de carbone, plus préférablement de 1 à 8 atomes de carbone. Des exemples, non limitatifs de groupes alkyle sont en particulier méthyle, éthyle, propyle, isopropyle, butyle, t-butyle, pentyle, hexyle, cyclohexyle.

Par « cycloalkyle », on entend un système carbocyclique saturé de 3 à 16, par exemple 3 à 7 atomes de carbone, de préférence 5 à 10 atomes de carbone, et plus préférablement 5 à 7 atomes de carbone et ayant 1, 2 ou 3 cycles. Des exemples, non limitatifs, de groupement cycloalkyle sont le cyclopropyle, le cyclopentyle, le cyclohéxyle et le cycloheptyle.

Par « *alcènyle* », on entend un groupe hydrocarboné aliphatique linéaire ou ramifié ayant au moins une double liaison carbone-carbone, et de 2 à 22 atomes de carbone, de préférence 2 à 12 atomes de carbone, plus préférablement de 2 à 8 atomes de carbone. Des exemples, non limitatifs, de groupes alkényles sont : l'éthényle, le propényle, le n-butényle, le n-pentényle, l'octényle et le décényle.

Par « *alcynyle* », on entend un groupe hydrocarboné aliphatique linéaire ou ramifié ayant au moins une triple liaison carbone-carbone, et de 2 à 6 atomes de carbone, de préférence 2 à 4 atomes de carbone. Des exemples, non limitatifs, de groupes alcynyles sont : l'éthynyle, le propynyle, le 2-butynyle, le 3-méthylbutynyle et le n-pentynyle.

Par « *hétéroalkyle* », on entend parmi les alkyles ci-dessus, un radical alkyle défini ci-dessus, dans lequel un ou plusieurs atomes de carbone est remplacé par un atome de soufre, d'oxygène ou d'azote.

Par « *aryle* », on entend, un système de groupement aromatique monocyclique ou multicyclique, comprenant au moins un noyau aromatique comprenant de 6 à 16 atomes de carbone, et de préférence de 6 à 10 atomes de carbone. Un exemple, non limitatif, de groupement aryle est le phényle.
Le groupement aryle peut être non substitué ou substitué avec 1, 2 3 ou 4 substituants choisis indépendamment les uns des autres. Un exemple, non limitatif, de groupement aryle substitué est l'hydroxyphényle.

Parmi les cycloalkyles, quand la chaîne du radical cycloalkyle est interrompue par un ou plusieurs hétéroatomes, il s'agit d'un « *hétérocycloalkyle* », par lesquels on entend un système non-aromatique saturé, monocyclique ou multicylique, comprenant de 3 à 16 (par exemple 3 à 7) atomes de carbone, de préférence 5 à 10 atomes de carbone, et plus préférablement 5 à 7 atomes de carbone et ayant 1, 2, 3 ou 4 cycles, dans lequel un ou plusieurs des atomes du système est un élément autre qu'un atome de carbone, tel que un atome de soufre, d'oxygène et/ou d'azote. Un exemple préféré d'hétérocycle selon la demande est pipéridyle.

Par « *arylalkyle* » ou « *aralkyle* », on entend aryle tel que défini ci-dessus fixé à un groupement alkyle tel que défini ci-dessus, le lien au groupement parent s'effectuant par le groupe alkyle. Un exemple, non limitatif, de groupement arylalkyle est le benzyle.

Par « *hétéroaryle* », on entend un système de 5 à 14, de préférence 5 à 10, noyaux aromatiques simples ou fusionnés, ces noyaux comprenant 1, 2 ou 3 hétéroatomes choisis indépendamment les uns des autres parmi O, S et N, sachant que les noyaux ne possèdent pas d'atomes d'oxygène ou de soufre adjacents. Un groupement hétéroaryle préféré contient de 5 à 6 atomes. Dans un autre mode de réalisation préféré, l'hétéroaryle est monocyclique. Le groupement hétéroaryle peut être non substitué ou substitué avec 1, 2 ou 3 substituants choisis indépendamment les uns des autres. Des exemples non limitatifs de groupements hétéroaryles sont: pyridyle, pyrazinyle, furanyle, thienyle, pyrimidinyle, isoxazolyle, isothiazolyle, oxazolyle, thiazolyle, pyrazolyle, furazanyle, pyrrolyle, pyrazolyle, triazolyle, 1,2, 4-thiadiazolyle, pyrazinyle, pyridazinyle, quinoxalinyle, phthalazinyle, imidazo [1,2- a] pyridinyle, imidazo [2, 1-b] thiazolyle, benzofurazanyle, indolyle, azaindolyle, benzimidazolyle, benzothienyle, quinolinyle, imidazolyle, thienopyridyle, quinazolinyle, thienopyrimidyle, pyrrolopyridyle, imidazopyridyle, isoquinolinyle, benzoazaindolyle, and benzothiazolyle. Des exemples préférés d'hétéroaryles monocycliques sont pyridyle, pyrazinyle, furanyle, thienyle, pyrimidinyle, isoxazolyle, isothiazolyle, oxazolyl, thiazolyle, pyrazolyle, furazanyle, pyrrolyle, pyrazolyle, triazolyle, pyrazinyle et pyridazinyle.

Par « *hétéroaralkyle* » ou « *hétéroarylalkyle* », on entend un groupe hétéroaryle tel que défini ci-dessus lié à un groupe alkyle tel que défini ci-dessus, le lien au groupement parent s'effectuant par le groupe alkyle.

Par « *groupe cycloamino* », on entend un groupe amino secondaire, système dialkylamino cyclique saturé de 3 à 10 (par exemple 3 à 7) atomes de carbone, de préférence 4 à 10 atomes de carbone, et plus préférablement 4 à 7 atomes de carbone et ayant 1, 2 ou 3 cycles. Un exemple, non limitatif, de groupe cycloamino est le groupe pipéridine.

Par « *aminoalkyle »,* on entend un groupe alkyle tel que défini ci-dessus, substitué par un ou plusieurs groupes NH₂. Un exemple non limitatif d'un tel groupe est le groupe -C₂H₄-NH₂.

Par « *substituant* », on entend un groupement, un éther, un hydroxyle, un carboxamide, un ester, une cétone, un aryle, un hétéroaryle, un cycloalkyle, une amine, une amine substituée, un alkyle linéaire ou ramifié, en particulier un alkyle de C₁ à C₆, un cyano, un nitro, un haloalkyle, un alkoxy, un carboxyalkyle, un mercapto, un sulfhydryle, un alkylamino, un dialkylamino, un sulfonyle et un sulfonamido. Dans un mode de réalisation particulier, lorsqu'un hétéroaryle est substitué, en particulier un hétéroaryle monocyclique, la substitution a lieu sur le ou les hétéroatome(s) du cycle. Alternativement ou en combinaison avec le mode de réalisation précédent, lorsque un hétéroaryle est substitué, en particulier un hétéroaryle monocyclique, la substitution a lieu sur le ou les atomes de carbone du cycle.
Dans la présente invention, les substituants sont de préférence hydroxyle, -O-alkyle, thiohydroxy ou -S-alkyle, dans le cadre de la définition de R₁. et aussi NH₂ ou -R-NH₂ où R est alkyle, dans le cadre de la définition de R₂ et R₃.

Par « *polyol du type glycérol* », parfois abrégé ici en « glycérol », on entend tout polyol comportant le motif -CH(OH)-CH₂OH. Ces diols peuvent avoir la formule (II) 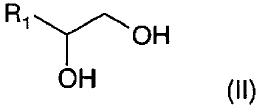 dans laquelle R₁ a la signification donnée ci-dessus.

Selon un aspect particulier de l'invention, le glycérol lui-même est utilisé, comme produit de départ et solvant. D'autres polyols, préférés eux aussi, sont le 1,2-propanediol et l'éthylène glycol, le digylcérol, le polyglycérol, l'amino-propanediol et les carbohydrates.

Par « *carbohydrate* », on entend un sucre monomère ou polymère, comme le glucose, le fructose ou le saccharose, cités à titre d'exemples non limitatifs, et plus généralement les mono- et poly-saccharides.

Dans la première variante, le milieu réactionnel pour réaliser la carbonatation du polyol de type glycérol comporte essentiellement ledit polyol, un carbonate choisi parmi le diéthylcarbonate, le diméthylcarbonate, le carbonate d'éthylène et le carbonate de propylène, et au moins un catalyseur basique, en milieu solvant.

Un des avantages du procédé selon l'invention résulte du fait que le polyol du type glycérol ou le glycérol peut être utilisé comme solvant dans le milieu réactionnel. Comme solvant, on peut toutefois utiliser un autre solvant que le glycérol ou du polyol du type glycérol impliqué, par exemple l'éthanol, le méthanol, l'eau ou un mélange de solvants.

Dans le second mode de réalisation, par « *carbonate cyclique à cinq chaînons* », on entend les carbonates cycliques de formule (IV) 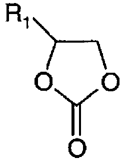 dans laquelle R₁ est tel que défini ci-dessus et substitue un cycle carbonate à cinq chaînons.
A titre d'exemple non limitatif, on peut citer le carbonate de glycérol, de propylène ou d'éthylène ou encore le monocarbonate de diglycérol, et plus généralement les carbonates de polyglycérol, y compris les polycarbonates cycliques à cinq chaînons, par exemple le bis-carbonate de diglycérol.

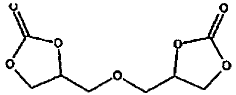

Par « *polycarbonates cycliques* à *cinq chaînons* », on entend donc les composés carbonate cycliques à cinq chaînons comportant au moins 2 cycles à cinq chaînons.
Par « *catalyseur* » dans le premier mode de réalisation, plus précisément « catalyseur basique », on entend selon l'invention tout catalyseur suffisamment basique pour déprotoner le polyol du type glycérol. Selon l'invention, on préfère les catalyseurs homogènes mais des catalyseurs hétérogènes peuvent toutefois être employés.

Comme catalyseur basique, on peut citer des bases minérales usuelles telles que KOH, NaOH, CsOH, et plus généralement les hydroxydes alcalins et alcalinoterreux, les hydroxydes d'ammonium, ou plus généralement encore R₄N⁺OH⁻, où R₄ représente un radical alkyle saturé en C₁-C₂₂. On peut citer aussi à titre de catalyseur basique une hydrotalcite réhydratée ou bien un liquide ionique basique, c'est-à-dire capable de déprotoner le polyol du type glycérol, par exemple porteur d'un groupe hydroxyle en contre-anion, comme de l'hydroxyde de méthylbutyllimidazolinium ou encore des résines polymères fortement basiques usuelles comme celles qui sont commercialisées sous la dénomination « Amberlyst » « A26OH » ou « A26CO₃ » par Rohm & Haas, « A260H » étant préférée. Selon un aspect préféré de l'invention, on utilise de la soude, KOH, CsOH, l'hydroxyde d'ammonium NH₄OH, ou l'hydroxyde de tétraméthyl ammonium.

Selon l'invention, la quantité de catalyseur peut varier de 0,2 à 20% molaire, de préférence 0,5 à 10 % molaire. De façon plus préférée, on utilise le catalyseur basique à raison d'environ 2 % molaire. Au-delà de 10% molaire, dans certains cas, des produits secondaires apparaissent lors de la réaction du carbonate de glycérol avec l'amine et le CS₂.

Le temps nécessaire à la réaction de carbonatation varie selon les produits de départ et les autres conditions réactionnelles, il peut varier de 2 minutes à environ 2h, de préférence 30 mn à 90 mn, de façon encore préférée environ 60 mn. Dans le premier mode de réalisation, le carbonate n'est pas isolé. Il s'agit d'une réaction one-pot.

La température du milieu réactionnel peut varier de la température ambiante (20°C) à 200°C, selon la nature des réactifs. En présence de carbonate d'éthyle, elle est de préférence de 100°C à 150 °C, de façon plus préférée de 120°C à 140°C, avec une préférence autour de 130°C.

A la suite de la carbonatation du polyol, on fait réagir, directement de préférence, le carbonate de glycérol intermédiaire avec l'amine et le CS₂. Selon l'invention, on introduit l'amine primaire ou secondaire et le sulfure de carbone dans le milieu réactionnel.

On détermine la fin de la réaction par exemple par la consommation des produits de départ ou le rendement maximal de la réaction. Pour récupérer le DTCG, on peut utiliser toute technique usuelle, notamment l'extraction liquide-liquide à l'aide d'un solvant adéquat, par exemple l'acétate d'éthyle. D'autres solvants peuvent cependant être employés, comme la méthylisobutylcétone ou le diéthyléther, ou encore CO₂ supercritique.

Pour la séparation, on peut aussi envisager la distillation, pour les produits suffisamment stables ou encore la précipitation et tout autre moyen adapté connu de l'homme de l'art et à sa portée.

Selon l'invention, on utilise une amine primaire ou secondaire, et toute amine est utilisable, dès lors qu'elle ou son sel d'ammonium est soluble dans le milieu réactionnel, ou qu'elle puisse y être solubilisée, ou encore qu'elle réagisse dans le milieu réactionnel, quelque soit son état. Les amines convenables selon l'invention peuvent être aromatiques, comme l'aniline, pourvu qu'elles soient primaires ou secondaires. Ces amines peuvent répondre à la formule (III) NHR₂R₃ dans laquelle R₂ et R₃ ont les significations données ci-dessus.
Selon un aspect particulier préféré de l'invention, les amines utilisées sont secondaires. Selon un autre aspect particulier de l'invention, on préfère les amines où R₂ et R₃ représentent une chaîne alkyle linéaire ou ramifiée en C₁-C₂₂. Selon un aspect particulier, R₂ et R₃ sont identiques.
Parmi les amines préférées selon l'invention, on peut citer les alkylamines ou dialkylamines en C₁ à C₂₂. On peut citer notamment la diéthylamine, la diméthylamine, la dipropylamine, la diisopropylamine, la diéthanolamine, la pipéridine, par exemple, ainsi que l'aniline.
Selon les conditions réactionnelles et les produits utilisés, l'étape de formation du DTCG à partir de l'introduction de l'amine peut varier de 1 et 48h, notamment entre 3 et 24h. Cette réaction peut notamment prendre environ 15h.
Selon les réactifs utilisés, la température peut varier entre 10°C et 100°C, par exemple entre 25 à 80 °C, avec une préférence pour une température de l'ordre de 60°C.
Du point de vue de la stoechiométrie, on préfère selon l'invention des conditions quasi-stoechiométriques azote/soufre. Toutefois, le rapport du polyol au carbonate peut aller de 1 à 10, de préférence 5 et du polyol à l'amine aller de 1 à 10, préférentiellement 5, et du polyol à CS₂ aller de 1 à 10, préférentiellement 5.

Dans le cas particulier du glycérol lui-même, l'invention offre la possibilité de synthétiser en une seule étape des dithiocarbamates de glycérol directement à partir du glycérol comme source de matière première. Ce procédé fait intervenir quatre réactifs chimiques (glycérol, diéthylcarbonate, CS₂ et une amine primaire ou secondaire) qui réagissent entre eux pour conduire aux dithiocarbamates de glycérol. Le schéma 4 ci-dessous présente un mécanisme de formation du dithiocarbamate de glycérol reposant sur la formation in situ du carbonate de glycérol.

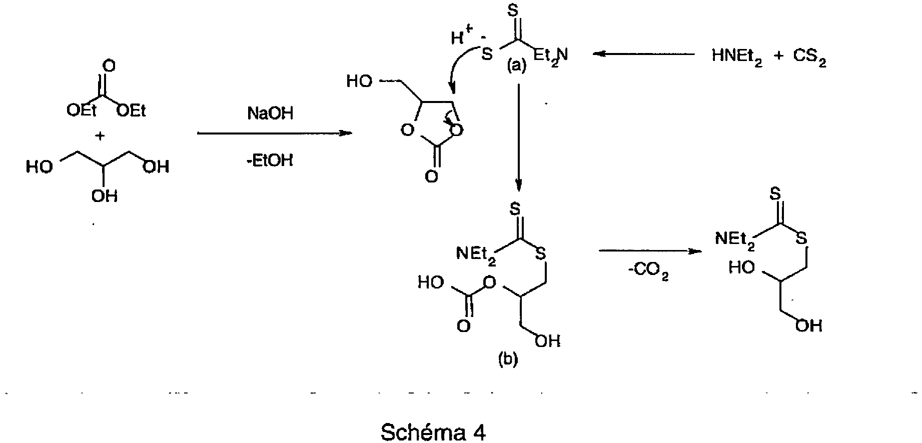

La première étape est la formation du carbonate de glycérol entre le diéthylcarbonate et le glycérol (réaction catalysée par NaOH). Le CS₂ et l'amine réagissent ensemble pour former un acide dithiocarbamique (a) qui vient ensuite s'additionner sur le carbonate de glycérol pour générer l'entité (b). Cette entité (b), instable, est décarboxylée pour conduire au dithiocarbamate de glycérol. Contrairement aux procédés chimiques usuels impliquant le glycérol, cette synthèse est non seulement chimiosélective mais également régiosélective. Cette sélectivité remarquable du procédé provient de la formation du carbonate de glycérol en tant qu'intermédiaire réactionnel. La présence d'un groupe -CH₂OH sur le carbonate de glycérol induit en effet un encombrement stérique important, responsable de la forte sélectivité de ce procédé.
Le sous-produit qu'est l'éthanol libéré lors de la réaction est facilement éliminé du milieu réactionnel, par distillation par exemple, récupéré et recyclé. Par ailleurs, le catalyseur homogène utilisé qu'est NaOH est couramment utilisé en industrie et a pu être recyclé au moins 6 fois sans perte notable d'activité.
De plus, du point de vue de la faisabilité industrielle, la transposition du procédé à l'échelle supérieure a été réalisée sans difficulté.
Il est ainsi apparu possible de mettre en oeuvre le procédé de l'invention à une échelle importante, comme il apparaîtra dans les exemples.

### Exemples Exemple 1 : Synthèse

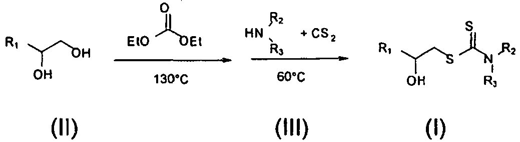

selon le mode opératoire général suivant :
Le polyol (84,5 mmol) a été mélangé avec 16,9 mmol de diéthylcarbonate en présence de 2% molaire de NaOH au cours de cette étape, l'éthanol est continuellement distillé et récupéré. Après 1h de réaction à 130°C, 16,9 mmol de CS₂ et 16,9 mmol de l'amine ont été ajoutées au milieu qui a été agité à 60°C pendant la durée H.

**Tableau 1**

| **Composé** | **R₁ polyol** | **R₂/R₃ Amine (*)** | **Durée H (h)** | **Produit** | **Rendement isolé (%)** |
|---|---|---|---|---|---|
| 1 | -CH₂OH | -Et | 15 | 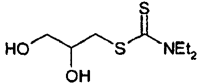 | 75 |
| 2 | -CH₂OH | -iPr | 24 | 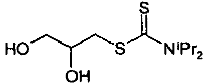 | 75 |
| 3 | -CH₂OH | * -(CH₂)₅* | 24 | 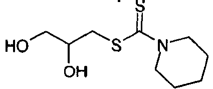 | 70 |
| 4 | -CH₂OH | -CH₂OH | 24 | 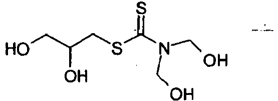 | 60 |
| 5 | -CH₂OH | -nBu | 24 | 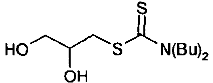 | 63 |
| 6 | -CH₃ | -Et | 24 | 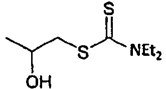 | 75 |
| 7 | -CH₃ | -(CH₂)₅ | 24 | 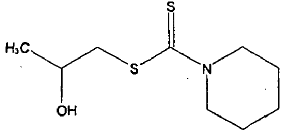 | 71 |
| 8 | -CH₃ | -iPr | 24 | 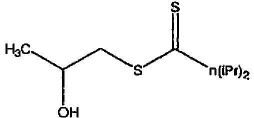 | 55 |
| 9 | -H | -Et | 24 | 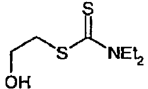 | 81 |
| 10 | -H | -iPr | 24 | 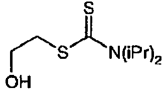 | 69 |
| 12 | -CH₂OH | -nOc* | 24 | 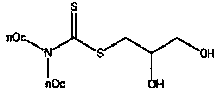 | 55 |

| | | | | | |
|---|---|---|---|---|---|
| R₂ = R₃ ou (*) -NR₂/R₃ = cycloamino | | | | | |

A l'issue de la durée H indiquée, le dithiocarbamate de glycérol correspondant a été obtenu avec le rendement indiqué au tableau 1 ci-dessus.
L'éthylène glycol et le 1,2-propandiol ont été utilisés à la place du glycérol avec succès conduisant aux dithiocarbamates correspondants (composés 6 à 10 et 12). Différentes amines ont été utilisées avec succès conduisant aux dithiocarbamates de glycérol correspondants.

### Exemple 2

### 2,3-dihydroxypropyl diéthyldithiocarbamate (composé 1)

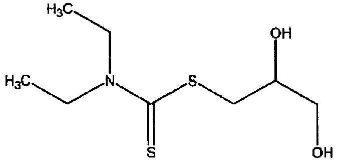

(huile brun jaune dans un mélange acétate d'éthyle/heptane = 3/7)
¹H NMR δ 1.27 (t, J = 7.1 Hz, 3H), 1.28 (t, J = 7.1Hz, 3H), 2.84 (s, 1H), 3.37 (s, 1 H), 3.51 (dd, J = 14.5 Hz, J = 6.5Hz, 1 H), 3.75 (q, J = 7.2 Hz, 2H), 3.78 (t, J = 7.1 Hz, 2H), 4.02 (t, J = 7.1 Hz, 2H);
¹³C NMR δ 11.3, 12.2, 40.8, 46.3, 48.9, 64.9, 69.8, 194.6 10.2, 10.4, 30.8, 31.0, 61.1, 62.4, 64.0, 64.2, 70.1, 70.2, 70.9, 71.1, 82.8, 84.5, 84.6, 126.7, 126.8, 127.6, 127.8, 128.4, 128.5, 141.9, 142.4.
IR (pur) 3365, 2975, 2932, 2873, 1631, 1488, 1442, 1416, 1379, 1354, 1300, 1268, 1204, 1142, 1090, 1067, 1005, 981, 915, 880, 832, 776 cm⁻¹;
LC.MS : [M+Na⁺] : m/z = 246

### Exemple 3

### 2,3-dihydroxypropyl pipéridine-1-carbodithioate (composé 3)

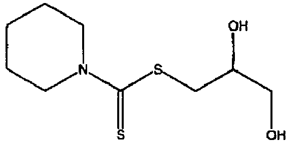

(huille jaune dans un mélange acétate d'éthyle/heptane = 3/7)
¹H NMR δ 1.71 (m, 6H), 3.55 (dd, J₁= 14Hz, J₂= 7Hz, 1H), 3.6-3.75 (m, 3H), 3.9-4 (m, 3H), 4.3 (m, 2H), 3.77-3.82 (m, 1 H), 4.12 (t, J = 6.7 Hz, 0.9 H), 4.30 (t, J = 6.8 Hz, 0.1 H), 7.21-7.34 (m, 5H);
¹³C NMR δ 24.2, 25.5, 26, 39.2, 51.7, 53.8, 64.6, 71.4, 195.7;
IR (pur) 3406, 2859, 1789, 1743, 1476, 1427, 1366, 1373, 1352, 1227, 1179, 1113, 1072, 1050, 1004, 975, 892, 853, 776, 711 cm⁻¹;
LC.MS : [M+Na⁺]: m/z = 258

### Exemple 4

### 2,3-dihydroxypropyl dibutyldithiocarbamate (composé 5)

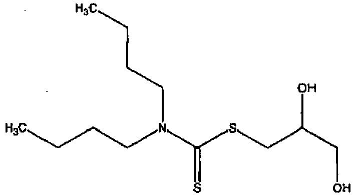

(huille jaune dans un mélange acétate d'éthyle/heptane = 3/7)
¹H NMR δ 0.95 (q, 6H), 1.38 (m, 4H), 1.7 (m, 4H), 3.55 (dd, J₁= 14Hz, J₂= 7Hz, 1 H), 3.6-3.7 (m, 5H), 3.95-4 (m, 3H);
IR (pur) 3363, 2958, 2931, 2872, 1788, 1642, 1484, 1456, 1413, 1367, 1289, 1249; 1217, 1184, 1144, 1091, 1030, 981, 944 930, 879, 731 cm⁻¹;
LC.MS : [M+Na⁺]: m/z = 302

### Exemple 5

### 2-hydroxypropyl pipéridine-1-carbodithioate (composé 7)

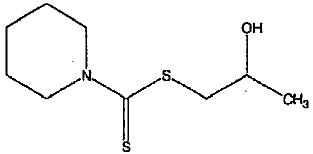

(huille jaune dans un mélange acétate d'éthyle /heptane = 3/7)
¹H NMR δ 1.25 (d, J = 4 Hz, 3H), 1.65 (m, 6H), 3.55 (dd, J = 14Hz, J = 7Hz, 1 H), 3.65 (dd, J= 14Hz, J = 4Hz, 1 H) 3.85 (m, 2H), 4 (m, 1 H), 4.2 (m, 2H);
¹³C NMR δ 22.52, 24.2, 25.5, 26, 44.9, 51.6, 53.5, 67.2, 195.9;
IR (pur) 3381, 2965, 2933, 2876, 1452, 1103, 1042, 727, 755, 700 cm⁻¹;
LC.MS : [M+Na⁺]: m/z = 228

### Exemple 6

### 2-hydroxypropyl diisopropyldithiocarbamate (composé 8)

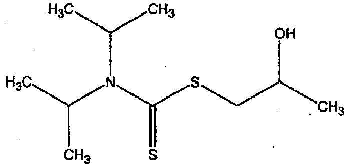

(huille jaune dans un mélange acétate d'éthyle/heptane = 3/7)
¹H NMR δ 1.25 (d, 3H), 1.2-2 (m, 12H), 2.94 (m, 1 H), 3.4 (m, 1 H), 3.7 (m, 1 H), 4.1 (m, 2H);
IR (pur) 3384, 2970, 2929, 2876, 1475, 1439, 1370, 1309, 1191, 1140, 1117, 1030, 960, 938, 896, 851, 777cm⁻¹;
LC.MS : [M+Na⁺]: m/z =258

### Exemple 7

### 2-hydroxyéthyl diéthyldithiocarbamate (composé 9)

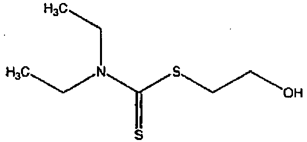

(huile jaune dans un mélange acétate d'éthyle/heptane = 3/7)
¹H NMR δ 1.24 (t, J = 7.2 Hz, 3H), 1.28 (t, J = 7.2Hz, 3H), 2.52 (s, 1 H), 3.55 (t, J = 6 Hz, 2H), 3.75 (q, J = 7.2 Hz, 2H), 3.86 (t, J = 6 Hz, 2H), 4.00 (q, J = 7.1 Hz, 2H); ¹³C NMR δ 11.6, 12.5, 39.12, 47.0, 49.9, 61.9, 195.6;
IR (pur) 3378, 2974, 2933, 2874, 1736, 1487, 1442, 1416, 1379, 1355, 1301, 1267, 1204, 1142, 1082, 1067, 1044, 1003, 980, 915, 832, 775cm⁻¹;
LC.MS : [M+Na⁺]: m/z = 216

### Exemple 8

### Le composé 12

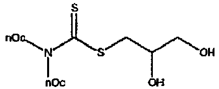

a été obtenu conformément au procédé de l'exemple 1.
De plus, on a réalisé la synthèse à une échelle bien supérieure, pour obtenir 1 kg du composé 12.
Le glycérol (7,3 mol) a été mélangé avec 14,6 mol de diéthylcarbonate en présence de 2% molaire de NaOH, au cours de cette étape, l'éthanol est continuellement distillé et récupéré. Après 2 heures de réaction à 130°C, 7,3 mol de CS₂ et 7,3 mol d'amine sont ajoutés au milieu qui est agité à 60°C pendant 72 heures. Le produit est ensuite purifié par colonne chromatographique avec l'heptane pur comme éluant.

### Exemple 9

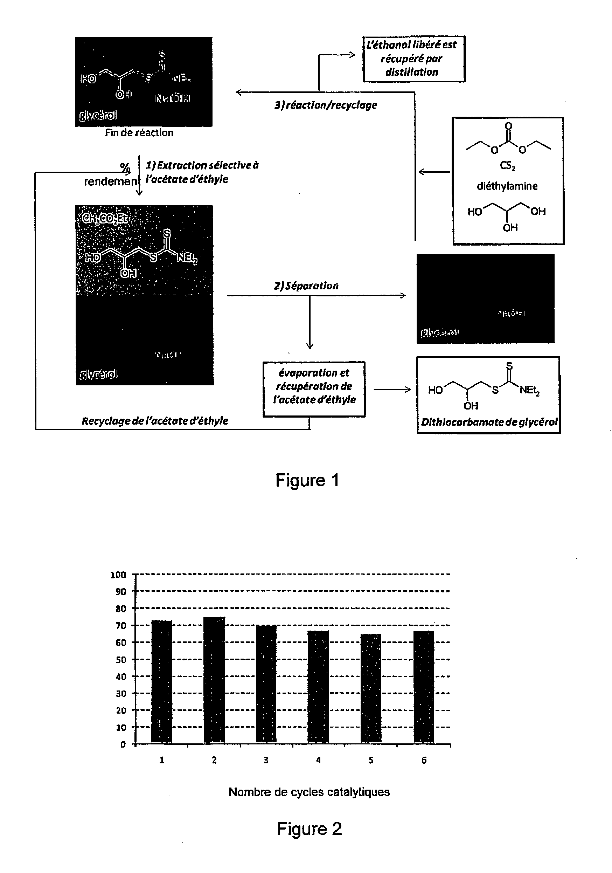

Pour montrer la possible limitation du coût économique et environnemental du procédé, le catalyseur (NaOH) a été recyclé ainsi que l'excès de glycérol utilisé. Une extraction liquide-liquide a été réalisée pour extraire les dithiocarbamates de glycérol de la phase glycérol avec de l'acétate d'éthyle. A partir de glycérol (84,5 mmol), de diéthylamine (16,9 mmol), de diéthyl carbonate (16,9 mmol), et de CS₂ (16,9 mmol). Après seulement deux extractions à l'acétate d'éthyle (4 x 30 ml), la totalité du dithiocarbamate de glycérol a été extraite (Figure 2).

Un titrage acido-basique de la phase glycérol a révélé que le catalyseur NaOH est entièrement resté dissous dans la phase glycérol. La phase glycérol/NaOH a été réutilisée. Ainsi, après rechargement de 16,9 mmol de glycérol, de diéthylamine, de CS₂ et de diéthyl carbonate, des rendements de 73% ont été à nouveau obtenus (Figure 2).
Comme cela apparaît à la figure 1, la phase glycérol/NaOH a pu être recyclée au moins 6 fois sans perte notable de rendement. L'acétate d'éthyle est également recyclé après chaque extraction.

### Exemple 10

Le procédé de l'exemple 1 a été transposé à l'échelle supérieure sans aucune difficulté et les dithiocarbamates de glycérol (10-15g) de l'exemple 1 ont tous été obtenus.

### Exemple 11

### Procédé avec carbonates cycliques à cinq chaînons

Dans l'éthanol à 60°C, les carbonates cycliques ci-dessous ont réagi selon le schéma 3, avec les amines indiquées, avec les rendements donnés ci-après au tableau Il à l'issue de la durée H.

| | | | | | |
|---|---|---|---|---|---|
| 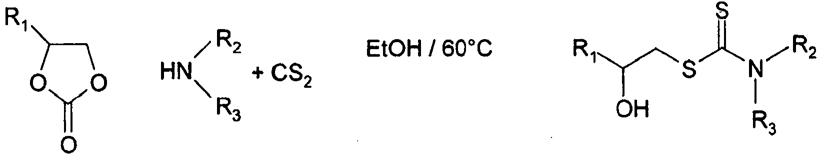 | | | | | |

| **Composé** | **R_{2/}R₃ Amine (*)** | **R₁ carbonate** | **Durée H (h)** | **Produit** | **Rdt isol. (%)** |
|---|---|---|---|---|---|
| 1 | -Et | -CH₂OH | 15 | 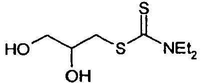 | 91 |
| 2 | -iPr | -CH₂OH | 24 | 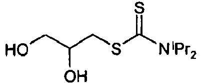 | 85 |
| 3 | -(CH₂)₅* | -CH₂OH | 24 | 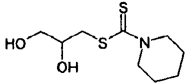 | 84 |
| 5 | -nBu | -CH₂OH | 24 | 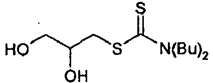 | 78 |
| 4 | -CH₂OH | -CH₂OH | 24 | 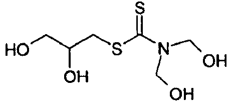 | 55 |
| 6 | -Et | -CH₃ | 24 | 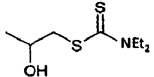 | 95 |
| 7 | -(CH₂)₅* | -CH₃ | 24 | 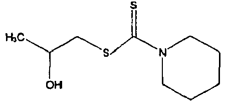 | 71 |
| 8 | -iPr | -CH₃ | 24 | 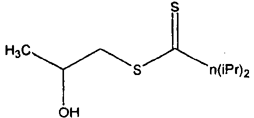 | 55 |
| 9 | -Et | -H | 24 | 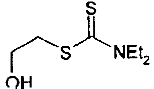 | 93 |
| 10 | -iPr | -H | 24 | 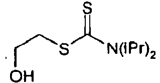 | 55 |
| 11 | -Et | - H₂OCH₂CHOHCH₂OH | 24 | 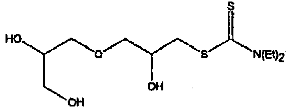 | 37 |

| | | | | | |
|---|---|---|---|---|---|
| * NR₂/R₃ = cyclomamino | | | | | |

**Tableau II**

### Exemple 12

De la même façon, on a mis en oeuvre les réactions suivantes

| | | | | |
|---|---|---|---|---|
| 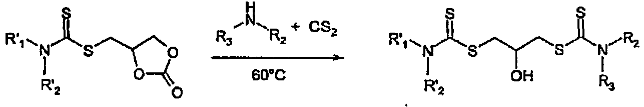 | | | | |

| **Composé** | **Amine** | **Durée (H)** | **Produit** | **Rendement isolé (%)** |
|---|---|---|---|---|
| 13 | -Et | 24 | 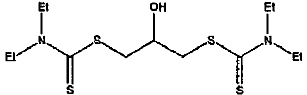 | 35 |
| 14 | -nBu | 40 | 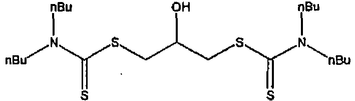 | 33 |
| 15 | -nOc | 48 | 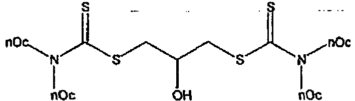 | 29 |

### Exemple 13

De façon similaire, avec deux équivalents amine et deux équivalents CS₂, on a mis en oeuvre les réactions suivantes

| | | | | |
|---|---|---|---|---|
| 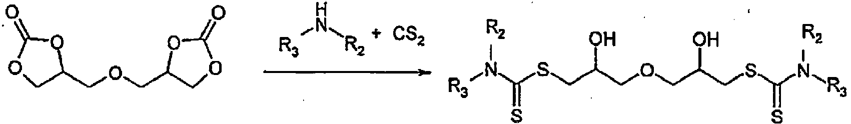 | | | | |

| **Composé** | **Amine** | **Durée (H)** | **Produit** | **Rendement isolé (%)** |
|---|---|---|---|---|
| 16 | -Et | 24 | 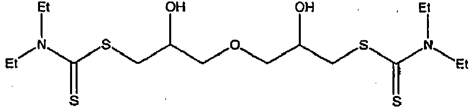 | 26 |
| 17 | -nOc | 40 | 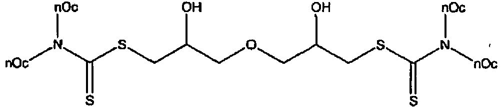 | 21 |

### Exemple 14

### 3-(2,3-dihydroxypropoxy)-2-hydroxypropyl diéthyldithiocarbamate (composé 11)

Selon le procédé de l'exemple 11, on a obtenu le composé du titre. 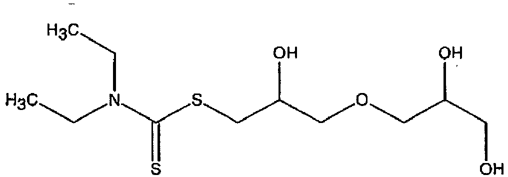 (huile jaune) 1 H NMR δ δ 1.20 (t, J = 7.1 Hz, 3H), 1.25 (t, J = 7.1 Hz, 3H), 3.3-3.5 (m, 6H), 3.55 (q, J = 6.5Hz, 1 H), 3.7 (m, 1H), 3.75 (q, J = 7.2 Hz, 2H), 3.82 (m, 1 H), 3.95 (q, J=7.2Hz, 2H), 4.05 (m, 1 H) ; 13C NMR δ 11.3, 14, 46.4, 49, 59.7, 63, 70.4, 72.9, 74.4, 82.1, 194.3

### REFERENCES

1. Rafin, E. Veignie, M. Sancholle, D. Postel, C. Len, P. Villa, G. Ronco, J. Agric. Food Chem., 2000, 48, 5283 ;
2. C. Len, D. Postel, G. Ronco, P. Villa, C. Goubert, E. Jeufrault, B. Mathon, H. Simon, J. Agric. Food Chem., 1997, 45, 3;
3. C. Len, A.-S. Boulogne-Merlot, D. Postel, G. Ronco, P. Villa, C. Goubert, E. Jeufrault, B. Mathon, H. Simon, J. Agric. Food Chem., 1996, 44, 2856;
4. C. Len, D. Postel, B. Meddah, P. Villa, G. Ronco, Phosphorus, Sulfur and Silicon, 2001, 173, 59;
5. C. Len, D. Postel, G. Ronco, P. Villa, J. Carbohydr. Chem., 1997, 16(7), 1029;
6. C. Len, D. Postel, G. Ronco, P. Villa, Phosphorus, Sulfur and Silicon, 1998, 133, 41.

## Revendications

1. Procédé de préparation de dithiocarbamates de glycérol (DTCGs) dans lequel, dans un milieu réactionnel comportant (a) un polyol du type glycérol associé à un carbonate choisi parmi le diéthylcarbonate, le diméthylcarbonate, le carbonate d'éthylène ou le carbonate de propylène en présence d'un catalyseur basique ou (b) un carbonate cyclique ou polycarbonate cyclique à cinq chaînons, en milieu solvant, on ajoute une amine primaire ou secondaire en présence de sulfure de carbone, et on récupère le DTCG formé.

2. Procédé selon la revendication 1, **caractérisé en ce que** le polyol du type glycérol qui est mis en présence du carbonate choisi parmi le diéthylcarbonate, le diméthylcarbonate, le carbonate d'éthylène ou le carbonate de propylène avant l'ajout de l'amine primaire ou secondaire a pour formule (II) 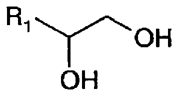 dans laquelle
- R₁ représente H ;
un radical alkyle en C₁-C₂₂, linéaire ou ramifié ou cycloalkyle en C₃-C₁₆, non-substitué ou substitué par un (ou plusieurs) groupe(s) hydroxy, -O-alkyle, thiohydroxy ou -S-alkyle, la chaîne du radical alkyle pouvant être interrompue par un ou plusieurs hétéroatomes O, N ou S ;
un radical alcènyle en C₂-C₂₂, linéaire ou ramifié, mono- ou poly-insaturé;
un radical alcynyle en C₂-C₆;
un radical aryle en C₆-C₁₀; ou
un radical arylalkyle en C₇ à C₂₂.

3. Procédé selon la revendication 1, **caractérisé en ce que** le carbonate cyclique à cinq chaînons a pour formule (IV) 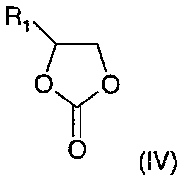 dans laquelle R₁ représente H ;
un radical alkyle en C₁-C₂₂, linéaire ou ramifié ou cycloalkyle en C₃-C₁₆, non-substitué ou substitué par un (ou plusieurs) groupe(s) hydroxy, -O-alkyle, thiohydroxy ou -S-alkyle, la chaîne du radical alkyle pouvant être interrompue par un ou plusieurs hétéroatomes O, N ou S ;
un radical alcènyle en C₂-C₂₂, linéaire ou ramifié, mono- ou poly-insaturé;
un radical alcynyle en C₂-C₆;
un radical aryle en C₆-C₁₀; ou
un radical arylalkyle en C₇ à C₂₂ ou

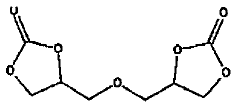

4. Procédé selon l'une des revendications 1 à 3, **caractérisé en ce que** le DTCG est de formule (I) R₁-C(OH)-CH₂-S-C-(S)-NR₂R₃
dans laquelle
- R₁ représente H ;
un radical alkyle en C₁-C₂₂, linéaire ou ramifié ou cycloalkyle en C₃-C₁₆, non-substitué ou substitué par un (ou plusieurs) groupe(s) hydroxy, -O-alkyle, thiohydroxy ou -S-alkyle, la chaîne du radical alkyle pouvant être interrompue par un ou plusieurs hétéroatomes O, N ou S ;
un radical alcènyle en C₂-C₂₂, linéaire ou ramifié, mono- ou poly-insaturé;
un radical alcynyle en C₂-C₆;
un radical aryle en C₆-C₁₀; ou
un radical arylalkyle en C₇ à C₂₂;
et
- R₂ et R₃, indépendamment l'un de l'autre, représentent H ;
un radical alkyle en C₁-C₂₂, linéaire ou ramifié ou cycloalkyle en C₃ à C₁₆ non-substitué ou substitué par un (ou plusieurs) groupe(s) hydroxy, -O-alkyle, thiohydroxy ou -S-alkyle, la chaîne du radical alkyle pouvant être interrompue par un ou plusieurs hétéroatomes O, N ou S ;
un radical alcènyle en C₂-C₂₂, linéaire ou ramifié, mono- ou poly-insaturé;
un radical alcynyle en C₂-C₆;
un radical aryle en C₆-C₁₆; ou
un radical arylalkyle en C₇ à C₂₂; ou
R₂ et R₃ ensemble avec l'atome d'azote auquel ils sont liés forment un groupe cycloamino,
ou de formule (I') 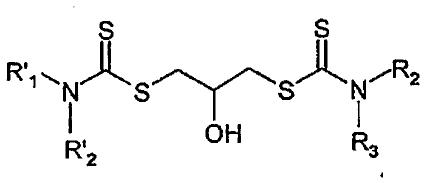 ou (I") 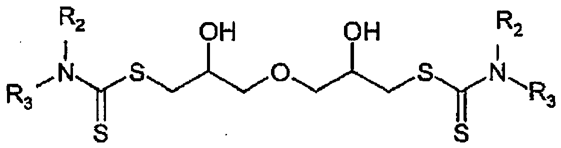 dans lesquelles R'₁ et R'₂ ont les mêmes significations que R₂ et R₃.

5. Procédé selon l'une des revendications 1 à 4, **caractérisé en ce que** l'amine est secondaire, de préférence une dialkylamine.

6. Procédé selon l'une des revendications 1 à 5, **caractérisé en ce que** l'amine est choisie parmi la diéthylamine, la diméthylamine, la dipropylamine, la diisopropylamine, la pipéridine, la diéthanolamine et l'aniline.

7. Procédé selon l'une des revendications 1, 2, ou 4 à 6, **caractérisé en ce que** le carbonate est du diéthylcarbonate.

8. Procédé selon l'une des revendications 1, 2 ou 4 à 7, **caractérisé en ce que** le polyol du type glycérol est choisi parmi le glycérol, le 1,2-propanediol et l'éthylène glycol.

9. Procédé selon l'une des revendications 1 à 8, **caractérisé en ce que** le catalyseur est choisi parmi KOH, NaOH, CsOH, l'hydroxyde d'ammonium ou de tétraalkylammonium, « Amberlyst A260H », une hydrotalcite réhydratée ou bien un liquide ionique basique, de préférence NaOH.

10. Procédé selon l'une des revendications 1 à 9, **caractérisé en ce que** la quantité de catalyseur va de 0,2 à 20% molaire, de préférence 0,5 à 10 % molaire, de façon plus préférée environ 2 % molaire.

11. Procédé selon l'une des revendications 1 à 10, **caractérisé en ce que** le carbonate cyclique à cinq chaînons est choisi parmi le carbonate de glycérol, le carbonate de propylène, le carbonate d'éthylène, le monocarbonate de glycérol et le bis-carbonate de diglycérol.

12. Procédé de préparation selon l'une des revendications 1 à 11, **caractérisé en ce que** le solvant du milieu réactionnel est un polyol du type glycérol.

13. Utilisation d'un polyol du type glycérol de formule (II) de la revendication 2 comme solvant dans un procédé selon l'une des revendications 1 à 12.

## Patentansprüche

1. Verfahren zur Zubereitung von Glycerin-Dithiocarbamaten (GDTC), wobei einem Reaktionsmedium, das (a) ein Polyol vom Typ Glycerin verbunden mit einem Carbonat gewählt aus Diethylcarbonat, Dimethylcarbonat, Ethylencarbonat oder Propylencarbonat in Gegenwart eines basischen Katalysators oder (b) ein fünfgliedriges zyklisches Carbonat oder zyklisches Polycarbonat in einem Lösemittelmedium umfasst, ein primäres oder sekundäres Amin in Gegenwart von Kohlenstoffdisulfid hinzugefügt und das gebildete GDTC gewonnen wird.

2. Verfahren nach Anspruch 1,
**dadurch gekennzeichnet, dass** das Polyol vom Typ Glycerin, das vor dem Hinzufügen des primären oder sekundären Amins mit Carbonat gewählt aus Diethylcarbonat, Dimethylcarbonat, Ethylencarbonat oder Propylencarbonat in Kontakt gebracht wird, die folgende Formel (II) aufweist 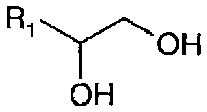 wobei
- R₁ für H steht;
einen geradkettigen oder verzweigten Alkyl-Rest mit 1 bis 22 Kohlenstoffatomen oder einen Cycloalkyl-Rest mit 3 bis 16 Kohlenstoffatomen, nicht substituiert oder substituiert durch eine (oder mehrere) Hydroxy-, -O-Alkyl-, Thiohydroxy- oder -S-Alkyl-Gruppe(n), wobei die Kette des Alkyl-Rests durch ein oder mehrere Heteroatome O, N oder S unterbrochen sein kann; einen geradkettigen oder verzweigten, einfach oder mehrfach ungesättigten Alkenyl-Rest mit 2 bis 22 Kohlenstoffatomen;
einen Alkinyl-Rest mit 2 bis 6 Kohlenstoffatomen;
einen Aryl-Rest mit 6 bis 10 Kohlenstoffatomen; oder
einen Arylalkyl-Rest mit 7 bis 22 Kohlenstoffatomen.

3. Verfahren nach Anspruch 1,
**dadurch gekennzeichnet, dass** das fünfgliedrige zyklische Carbonat die folgende Formel (IV) aufweist: 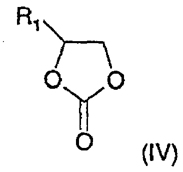 wobei R₁ für H steht;
einen geradkettigen oder verzweigten Alkyl-Rest mit 1 bis 22 Kohlenstoffatomen oder einen Cycloalkyl-Rest mit 3 bis 16 Kohlenstoffatomen, nicht substituiert oder substituiert durch eine (oder mehrere) Hydroxy-, -O-Alkyl-, Thiohydroxy- oder -S-Alkyl-Gruppe(n), wobei die Kette des Alkyl-Rests durch ein oder mehrere Heteroatome O, N oder S unterbrochen sein kann;
einen geradkettigen oder verzweigten, einfach oder mehrfach ungesättigten Alkenyl-Rest mit 2 bis 22 Kohlenstoffatomen;
einen Alkinyl-Rest mit 2 bis 6 Kohlenstoffatomen;
einen Aryl-Rest mit 6 bis 10 Kohlenstoffatomen; oder
einen Arylalkyl-Rest mit 7 bis 22 Kohlenstoffatomen oder

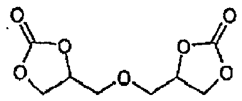

4. Verfahren nach einem der Ansprüche 1 bis 3,
**dadurch gekennzeichnet, dass** das GDTC die Formel (I) R₁-C(OH)-CH₂-S-C-(S)-NR₂R₃ aufweist, wobei
- R₁ für H steht;
einen geradkettigen oder verzweigten Alkyl-Rest mit 1 bis 22 Kohlenstoffatomen oder einen Cycloalkyl-Rest mit 3 bis 16 Kohlenstoffatomen, nicht substituiert oder substituiert durch eine (oder mehrere) Hydroxy-, -O-Alkyl-, Thiohydroxy- oder -S-Alkyl-Gruppe(n), wobei die Kette des Alkyl-Rests durch ein oder mehrere Heteroatome O, N oder S unterbrochen sein kann;
einen geradkettigen oder verzweigten, einfach oder mehrfach ungesättigten Alkenyl-Rest mit 2 bis 22 Kohlenstoffatomen;
einen Alkinyl-Rest mit 2 bis 6 Kohlenstoffatomen;
einen Aryl-Rest mit 6 bis 10 Kohlenstoffatomen; oder
einen Arylalkyl-Rest mit 7 bis 22 Kohlenstoffatomen;
und
- R₂ und R₃ jeweils unabhängig voneinander für H steht;
einen geradkettigen oder verzweigten Alkyl-Rest mit 1 bis 22 Kohlenstoffatomen oder einen Cycloalkyl-Rest mit 3 bis 16 Kohlenstoffatomen, nicht substituiert oder substituiert durch eine (oder mehrere) Hydroxy-, -O-Alkyl-, Thiohydroxy- oder -S-Alkyl-Gruppe(n), wobei die Kette des Alkyl-Rests durch ein oder mehrere Heteroatome O, N oder S unterbrochen sein kann;
einen geradkettigen oder verzweigten, einfach oder mehrfach ungesättigten Alkenyl-Rest mit 2 bis 22 Kohlenstoffatomen;
einen Alkinyl-Rest mit 2 bis 6 Kohlenstoffatomen;
einen Aryl-Rest mit 6 bis 16 Kohlenstoffatomen; oder
einen Arylalkyl-Rest mit 7 bis 22 Kohlenstoffatomen; oder
R₂ und R₃ zusammen mit dem Stickstoffatom, an das sie gebunden sind, eine Cycloamino-Gruppe bilden,
oder die Formel (I') aufweisen 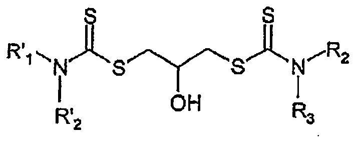 oder (I") 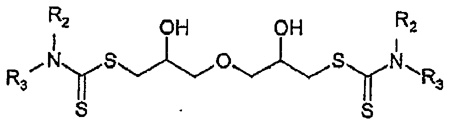 wobei R'₁ und R'₂ die gleichen Bedeutungen haben wie R₂ und R₃.

5. Verfahren nach einem der Ansprüche 1 bis 4,
**dadurch gekennzeichnet, dass** das Amin sekundär ist, vorzugsweise ein Dialkylamin.

6. Verfahren nach einem der Ansprüche 1 bis 5,
**dadurch gekennzeichnet, dass** das Amin gewählt ist aus Diethylamin, Dimethylamin, Dipropylamin, Diisopropylamin, Piperidin, Diethanolamin und Anilin.

7. Verfahren nach einem der Ansprüche 1, 2 oder 4 bis 6,
**dadurch gekennzeichnet, dass** das Carbonat Diethylcarbonat ist.

8. Verfahren nach einem der Ansprüche 1, 2 oder 4 bis 7,
**dadurch gekennzeichnet, dass** das Polyol vom Typ Glycerin gewählt ist aus Glycerin, 1,2-Propandiol und Ethylenglykol.

9. Verfahren nach einem der Ansprüche 1 bis 8,
**dadurch gekennzeichnet, dass** der Katalysator gewählt ist aus KOH, NaOH, Ammonium- oder Tetraalkylammoniumhydroxid, "Amberlyst A260H", rehydratisiertem Hydrotalcit oder auch einer basischen ionischen Flüssigkeit, vorzugsweise NaOH.

10. Verfahren nach einem der Ansprüche 1 bis 9,
**dadurch gekennzeichnet, dass** die Katalysatormenge 0,2 bis 20 Mol.-%, vorzugsweise 0,5 bis 10 Mol.-%, besonders bevorzugt etwa 2 Mol.-% beträgt.

11. Verfahren nach einem der Ansprüche 1 bis 10,
**dadurch gekennzeichnet, dass** das fünfgliedrige zyklische Carbonat gewählt ist aus Glycerincarbonat, Propylencarbonat, Ethylencarbonat, Glycerinmonocarbonat und Diglycerinbicarbonat.

12. Zubereitungsverfahren nach einem der Ansprüche 1 bis 11,
**dadurch gekennzeichnet, dass** das Lösemittel des Reaktionsmediums ein Polyol vom Typ Glycerin ist.

13. Verwendung eines Polyols vom Typ Glycerin mit der Formel (II) des Anspruchs 2 als Lösemittel in einem Verfahren nach einem der Ansprüche 1 bis 12.

## Claims

1. A method for the preparation of glycerol dithiocarbamates (GDTCs) in which a reaction medium comprising (a) a glycerol type polyol associated with a carbonate selected from diethyl carbonate, dimethyl carbonate, ethylene carbonate and propylene carbonate in the presence of a basic catalyst, or (b) a cyclic carbonate or cyclic polycarbonate comprising five ring members, in a solvent medium, is supplemented with a primary or secondary amine in the presence of carbon disulphide, and the GDTC formed is recovered.

2. A method according to claim 1, **characterized in that** the glycerol type polyol which is brought into the presence of the carbonate selected from diethyl carbonate, dimethyl carbonate, ethylene carbonate and propylene carbonate before supplementing with the primary or secondary amine has the following formula (II): 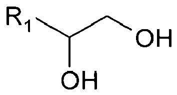 in which
- R₁ represents H;
a C₁-C₂₂ alkyl radical, linear or branched or a C₃-C₁₆ cyclo-alkyl radical, not substituted or substituted with one (or more) hydroxy, -O-alkyl, thiohydroxy or -S-alkyl group(s), the chain of the alkyl radical possibly being interrupted by one or more O, N or S heteroatoms;
a linear or branched, mono- or poly-unsaturated C₂-C₂₂ alkenyl radical;
a C₂-C₆ alkynyl radical;
a C₆-C₁₀ aryl radical; or
a C₇ to C₂₂ arylalkyl radical.

3. A method according to claim 1, **characterized in that** the cyclic carbonate comprising five ring members has the following formula (IV): 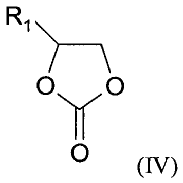 in which R₁ represents H;
a C₁-C₂₂ alkyl radical, linear or branched or a C₃-C₁₆ cyclo-alkyl radical, not substituted or substituted with one (or more) hydroxy, -O-alkyl, thiohydroxy or -S-alkyl group(s), the chain of the alkyl radical possibly being interrupted by one or more O, N or S heteroatoms;
a linear or branched, mono- or poly-unsaturated C₂-C₂₂ alkenyl radical;
a C₂-C₆ alkynyl radical;
a C₆-C₁₀ aryl radical; or
a C₇ to C₂₂ arylalkyl radical; or

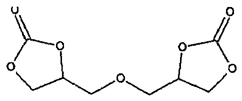

4. A method according to one of claims 1 to 3, **characterized in that** the GDTC has formula (I) R₁-C(OH)-CH₂-S-C-(S)-NR₂R₃
in which
- R₁ represents H;
a C₁-C₂₂ alkyl radical, linear or branched or a C₃-C₁₆ cyclo-alkyl radical, not substituted or substituted with one (or more) hydroxy, -O-alkyl, thiohydroxy or -S-alkyl group(s), the chain of the alkyl radical possibly being interrupted by one or more O, N or S heteroatoms;
a C₂-C₂₂ alkenyl radical, linear or branched, mono- or poly-unsaturated;
a C₂-C₆ alkynyl radical;
a C₆-C₁₀ aryl radical; or
a C₇ to C₂₂ arylalkyl radical;
and
- R₂ and R₃, independently of each other, represent H;
a C₁-C₂₂ alkyl radical, linear or branched or a C₃-C₁₆ cyclo-alkyl radical not substituted or substituted with one (or more) hydroxy, -O-alkyl, thiohydroxy or -S-alkyl group(s), the chain of the alkyl radical possibly being interrupted by one or more O, N or S heteroatoms;
a linear or branched, mono- or poly-unsaturated C₂-C₂₂ alkenyl radical;
a C₂-C₆ alkynyl radical;
a C₆-C₁₆ aryl radical; or
a C₇ to C₂₂ arylalkyl radical; or
R₂ and R₃ together with the nitrogen atom to which they are bonded form a cycloamino group;
or with formula (I'): 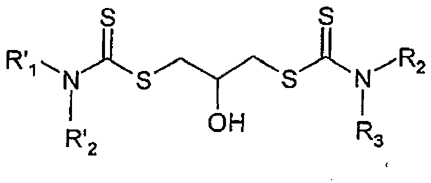 or (I"): 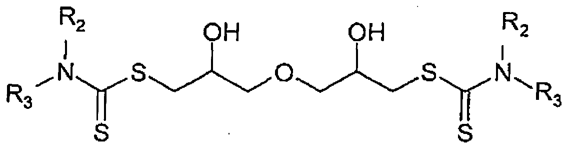 in which R'₁ and R'₂ have the same meanings as R₂ and R₃.

5. A method according to one of claims 1 to 4, **characterized in that** the amine is a secondary amine, preferably a dialkylamine.

6. A method according to one of claims 1 to 5, **characterized in that** the amine is selected from diethylamine, dimethylamine, dipropylamine, diisopropylamine, piperidine, diethanolamine and aniline.

7. A method according to one of claims 1, 2 or 4 to 6, **characterized in that** the carbonate is diethyl carbonate.

8. A method according to one of claims 1, 2 or 4 to 7, **characterized in that** the glycerol type polyol is selected from glycerol, 1,2-propanediol and ethylene glycol.

9. A method according to one of claims 1 to 8, **characterized in that** the catalyst is selected from KOH, NaOH, CsOH, ammonium or tetraalkylammonium hydroxide, "Amberlyst A260H", a rehydrated hydrotalcite or a basic ionic liquid, preferably NaOH.

10. A method according to one of claims 1 to 9, **characterized in that** the quantity of catalyst is in the range 0.2% to 20% molar, preferably in the range 0.5% to 10% molar, more preferably approximately 2% molar.

11. A method according to one of claims 1 to 10, **characterized in that** the cyclic carbonate comprising five ring members is selected from glycerol carbonate, propylene carbonate, ethylene carbonate, glycerol monocarbonate and diglycerol bis-carbonate.

12. A method according to one of claims 1 to 11, **characterized in that** the solvent for the reaction medium is a glycerol type polyol.

13. Use of a glycerol type polyol with formula (II) in claim 2 as a solvent in a method according to one of claims 1 to 12.
